# EUROPEAN PATENT APPLICATION

(11) **EP 0 877 082 A1**
(43) Date of publication of application: **11.11.1998**
(21) Application number: 97201281.9
(22) Date of filing: 05.05.1997
(51) Int. Cl.: C12N 15/00, A01K 67/027, C12N 15/01

(54) **Gene targeting and sibling-selection to generate mutant individuals**

(71) Applicant: Vereniging Het Nederlands Kanker Instituut, Nl-1066 CX Amsterdam (NL)
(72) Inventor: The designation of the inventor has not yet been filed
(74) Representative: Smulders, Theodorus A.H.J., Ir.

(57) **Abstract**

The invention relates to a method to generate defined mutant individuals from parent organisms with a known gene. In particular the invention relates to a method to generate defined mutant individuals from multi-cellular parent organisms where homologous recombination is not easily applicable e.g. because embryonal stem cells or oocytes cannot easily be handled. In addition, the invention relates to a method wherein the parent organism is a uni-cellular organism such as a bacterium or other micro-organism or a virus, especially when these micro-organisms cannot easily be transfected or transduced and are thus less accessible to homologous recombination techniques.
The mutant individual originate from a parent organism that is mutagenized and the mutant individual is selected by sibling-selection via amplification of or hybridisation with at least a part of the known but now mutagenized gene of offspring of the mutagenized parent organism.

## Description

The invention relates to a method to generate defined mutant individuals from parent organisms with a known gene. In particular the invention relates to a method to generate defined mutant individuals from multi-cellular parent organisms where homologous recombination is not easily applicable (e.g. because embryonal stem cells or oocytes cannot easily be handled). In addition, the invention relates to a method wherein the parent organism is a uni-cellular organism such as a bacterium or other micro-organism or a virus, especially when these micro-organisms cannot easily be transfected or transduced and are thus less accessible to homologous recombination techniques. The mutant individual originates from parent organisms that are mutagenized and the mutant individual is selected by sibling-selection of offspring of the mutagenized parent organism.

Modern biotechnological methods will in all likelihood provide within the foreseeable future vast stretches or even entire genome sequences and thus insight in the genotype of an ever widening array of micro-organisms or unicellular organisms, animals and plants. In addition, classical biology can often provide a very detailed description of the organism's morphology, development, and physiology, and how and in which patterns these phenotypic characteristics are inherited. Thus, the complete phenotype and genotype of various micro-organisms, animals and plants will increasingly become known.

However, what is then still not known is how genotype determines phenotype; to study this, one needs to establish connections between genomic sequence and phenotype. Much has been done by classic or forward genetics to study this relationship. Mutagenesis experiments have identified loci involved in a specific trait. Many of these loci have already been defined at the molecular level, and the genome sequence will certainly aid in the identification of many more. However, the reciprocal approach, namely reverse genetics, becomes by default far more important when a genome sequence is already determined but when the function or importance of the determined sequence is not yet known. Given the sequence of a gene of which nothing else is known, how can the function of that gene be determined?
One can study a gene's function by several approaches of which some are listed below.
1. Comparison of a gene sequence with those of other genes can indicate possible functions. In a few cases similarity to a gene of known function can identify a gene as a member of a gene family.
2. The tissue- and stage-specific expression pattern can give a hint (but no more than a hint) of the gene's function.
3. The ultimate approach is to alter the sequence of the known gene and study the effect on phenotype. The alteration can be complete inactivation, partial inactivation, or a more specific change. These types of experiments could be referred to as reverse genetics in a stricter sense.

To alter an endogenous gene by reverse genetics one has to target the known gene with the unknown function and alter the gene so that viable mutant individuals arise, which may be functionally different from the parent organism. A widely known method to alter genes is by homologous recombination techniques, in which one modifies by recombinant DNA technology the isolated gene of interest, after which this gene is re-introduced in cells of the organism which subsequently can give rise to sufficient numbers of offspring of said altered organism. The altered gene replaces the parent gene in the genome of the treated cell by homologous recombination events. This technique of homologous recombination generally works very well for unicellular organisms, or for multi-cellular organisms that can originate from single cells (such as plants), and also for organisms smaller than cells, such as viruses or plasmids, that replicate in cells.

For micro-organisms (unicellular organisms and viruses and plasmids), that generally can be easily cultivated in cell-culture, a wide array of transfection or transduction techniques, such as electroporation, has been described by which the altered gene or other foreign DNA can be introduced in the cell, after which homologous recombination can occur. After homologous recombination has been effected, it is easy to select for the transgenic micro-organisms that contain the altered gene, and it is easy to multiply the offspring to sufficiently large numbers to study by reverse genetics the relationships between the gene of interest and its resulting genotypes. From larger animals one can isolate from the gonades the embryonic stem cells or oocytes in which foreign DNA can be introduced via micro-injection techniques. Especially with those (experimental) animals for which techniques allowing cultivation, handling and maybe even cloning of embryonic stem cells and/or oocytes are available, micro-injection techniques can be used with good results in the production of transgenic and mutant animals, which can then be studied by reverse genetics.

However, homologous recombination, albeit relatively simple to employ, cannot easily be applied rapidly on a large scale. Fast mutagenization of micro-organisms and e.g. cells derived of embryonic stem cell lines, would allow a preselection of micro-organisms and stem cells with a known defect in a gene. In a second step the mutagenized cell or micro-organism can again be replicated and used as substrate for subsequent transgenesis of the now defunct gene.

Also, for a large group of animals, such as insects as *Drosophila melanogaster*, various nematodes such as *Caenorhabditis elegans*, and plants, such as *Arabidopsis thaliana*, these techniques for reverse genetics to produce mutant individuals that are deliberately mutagenized in a previously determined known gene are not available, or are to cumbersome to be of practical use. Yet, it would be extremely practical and valuable to study exactly these types of animals or plants by reverse genetics. They are small enough to be housed in large populations, yet large enough to harbour complex organs and interplaying organ systems. They display developmental processes that resemble those of larger animals and plants but can be bred with small generation intervals. All properties which greatly facilitate studying relationships between genotype and phenotype. However, for example, the strategy of targeted gene interruption by homologous recombination with transgenic DNA has been tried for organisms such as nematodes and fruit flies, but has never worked satisfactorily.

For example with nematodes, it is possible that the frequency with which naked microinjected DNA integrates homologously into the nematode genome is not very different from that in other species, but the absolute number of progeny that have been exposed to the transgenic DNA is limited by the number of injections one can perform, which makes the production of transgenes by homologous recombination in these organisms a cumbersome affair. Nevertheless, a few cases of success have been reported, using either plasmid DNA or oligonucleotides. An alternative for homologous recombination is the use of antisense RNA. It has been shown that one can largely inactivate the function of a gene by introduction of a transgene that carries a gene segment in inverted orientation with respect to the promoter. An advantage of this approach is that one can in principle aim to inactivate gene function in a subset of cells only, by the choice of a specific promoter. Two limitations should also be considered: it is possible that gene inactivation is lethal, which would result in inability to generate the transgenic line (with a knockout one can always make the mutants first, as heterozygote, and test the phenotype of the homozygote subsequently). A more important limitation is that the absence of a phenotype does not mean much: either it could be the real effect of loss of gene function, or it could reflect insufficient effect of the transgene. A third strategy is not detailed here, as it is not strictly reverse genetics: one can make an assumption about phenotype (e.g., that a gene is essential for viability) and use that to search for mutants among a mutagenized population. For example, one can precomplement with an extrachromosomal transgene that contains the gene of interest in many copies, mutagenize the strain, and look for progeny that have become obligatory transgenics (and die when the transgene is lost). A fourth strategy makes use of insertion of Tc1 transposons in the genome of the host. Insertion of Tcl into a gene can interrupt that gene and therefore inactivate it, but insertion into introns may leave gene function intact. Such insertions that do not result in loss of gene function may nevertheless be used as a first step toward gene inactivation, as progeny may be selected in which the transposon plus part of the interrupted gene sequences have been deleted and lost. The Tc1 transposon method is derived from a method that has found some use in the fruitfly, and is also applicable in other organisms, such as nematodes, although its application there has thusfar been limited by the logistics of the procedure and the strong regional preferences for P transposon insertion. A strain that contains several copies of the Tcl transposon and is permissive for Tcl transposition is taken as a source of new "spontaneous" insertion alleles. However, even target selected gene inactivation via transposon insertion followed by selection and isolation of deletion derivatives is rather cumbersome, and uses two steps and a fairly long road towards the generation of transgenic individuals in which a gene has been modified or inactivated in such a way as to allow for the generation of offspring and study genotype-phenotype relationships via reverse genetics. Specifically, the second step of the method depends on culturing of the organism or a cell line of the organism with a transposon insertion in the target gene, and is thus not easily scalable.

The invention provides a method to generate defined mutant individuals from parent organisms with a known gene. In particular, the invention now provides a method to generate in a method defined mutant individuals from multi-cellular parent organisms. In addition, the invention provides a method wherein the parent organism is a uni-cellular organism such as a bacterium or other micro-organism or a virus, especially when these micro-organisms cannot easily be transfected or transduced and are thus less accessible to homologous recombination techniques.
Furthermore, the invention provides a method to mutagenize embryonic stem cells capable of replicating in culture, possibly derived of embryonic stem cell lines. The individually mutagenized stem cell can again be replicated and optionally used to generate a whole individual.
A method provided by the invention is aimed at the isolation of an individual organism in which a previously known gene of interest has been inactivated by at least one deletion. It requires knowledge of a DNA or RNA sequence of at least a part of the gene of interest. Another requirement is that it is possible to grow sufficiently large numbers of the organism, such that after a random mutagenesis (see below) there is a reasonable chance that one or a few of the organisms have undergone a deletion in the gene of interest and have produced offspring with the wanted mutations, and such that the offspring can be sorted out by a "sibling-selection" protocol. Gene inactivation or random mutagenesis can be achieved with chemical or physical methods, according to protocols described in the literature and known in the art. Examples of such chemicals are EMS (ethyl-methane-sulphonate) for which it is estimated that up to 10% of all mutations it generates are deletions. Also used can be combined TMP/UV (TMP = trimethyl psoralene) treatment which is chemical mutagenesis with in this case psoralene followed by UV irradiation, which treatment by itself is a less powerful mutagenic treatment than the treatment with EMS, but which among the mutants it generates shows a higher proportion of deletions. However, many more protocols to generate mutagenised genomes are known in the art. Offspring selection via sibling-selection is a widely practised method by animal and plant breeders, where genotypic and phenotypic characteristics of individuals are determined by testing the siblings of these individuals.

A method provided by the invention to test the offspring is amplification of the remaining part of the deleted gene of interest by amplification of said gene parts, using primers selected from sequences flanking core sequences of the gene. Primer selection is in itself already a means to deletion selection, by varying the selected primers one can vary the amplified fragment, and thus test for differing mutant individuals. A possible amplification method is polymerase chain reaction, however, other nucleic acid amplification methods are known in the art. By varying elongation times it is also possible to test for mutant individuals with deleted gene parts of varying lengths. Another method to test the offspring uses hybridisation with those parts of the gene or sequences flanking the gene that have remained in the offspring. Again, by selecting various known nucleic acid probes, it is possible to select for differing mutants. Suitable hybridisation methods are known in the field, such as Southern Blotting or *in situ* hybridisation.

The methods provided by the invention are applicable to a wide array of micro-organisms and to multi-cellular organisms, be it from the plant or animal kingdom. Examples could be baceria and viruses and insects such as *Drosophila melanogaster*, nematodes such as *Caenorhabditis elegans*, and plants, such as *Arabidopsis thaliana*, stem cells that can be cultured but other micro-organisms or multi-cellular organisms are also known to those with ordinary skills in the field. Experimental part

*Caenorhabditis elegans* is in all likelihood the first metazoan animal whose entire genome sequence will be determined (Wilson et al, 1994). Therefore the method was initially developed with *Caenorhabditis elegans*. The standard laboratory strain Bristol N2 was used. This was mutagenized with chemicals, according to protocols described in the literature (e.g. Anderson, 1995) . We used EMS, for which it is estimated that up to 10% of all mutations it generates are deletions. We also used TMP/UV (psoralene with UV irradiation), which by itself is a legs powerful mutagen than EMS, but which among the mutants it generates shows a higher proportion of deletions. After mutagenesis single young adult hermaphrodites were cloned out on 10 cm NGM plates; when the plates were grown full, the animals were harvested and split in three samples: two were frozen for survival (using a method published previously, see Plasterk, 1995 for details), and one was stored for DNA isolation. We counted that mutagenized parents on average produced 50-150 F1 progeny; since each is diploid and expected to be heterozygous for every mutation caused by the chemical treatment, we expect each culture to contain between 100 and 300 independent mutagenized genomes. We generated a library of approximately 5000 cultures (from the EMS mutagenesis) and 3000 (from the TMP/UV mutagenesis); in total presumably containing approximately one million mutagenized genomes. All preparations were frozen in tubes stored in a standard microtiter array format, 96 tubes per rack. Then from each preparation to be analyzed for its DNA contents, the following was done: superfluous buffer, previously used to wash worms from their culturing plates, was removed, and to the wet pellets we added 500 microliter of a lysis solution (see Plasterk 1995). After lysis at 65°C, the crude lysates could be stored again in a frozen state. For subsequent analysis we pooled 100 microliter of each lysate from a 96 tube rack into one larger tube, and purified DNA (see Plasterk 1995 for details on the DNA purification). We then determined experimentally which dilution of these DNA mixtures could best be used; a too high concentration results in loss of all PCR product signals, while a too low concentration reduces the chances of detecting deletion products.

The screen for deletion products can be done by using amplification methods such as polymerase chain reaction (PCR) or hybridisation. In this case we performed two rounds of PCR, using nested primers. The primers were around 20 nucleotides in length, and the outer primer pair was chosen just flanking the inner primer pair. The prime sites are approximately 4-5 kilo base pairs apart, and point towards each other. PCR was done in a 96 well microtiter format, as described (Plasterk 1995 in: Meth. in Cell Biology Academic Press Vol. 48, p 59-80); the elongation times were slightly shortened with respect to the published method, to enhance the selective pressure for deletions. The basis for the selective visualization of deletions lies in the fact that the prime sites are far apart, so that the full length product corresponding to the wild type genome sequence is barely visible on an ethidium bromide stained agarose gel. A product derived from a genome in which a deletion has occurred between the left and right prime sites, will be amplified more efficiently. Since this gain in efficiency is found in each cycle of the chain reaction, there is an immense bias to visualize deletions. Enlarging or shortening elongation times allows for the selection of mutants with deletions of varous sizes in the known gene. In fact we have found (results not shown) that it is possible to visualize deletions even when these are present in less than 0.1% of the genomes in a DNA sample.

All positive samples were tested again in several fold (usually 12), and compared to negative neighbouring samples. When a positive signal was confirmed, the corresponding culture was analyzed further: it was split into several pools of reduced complexity. In fact we usually prefer to initiate pools of different complexities: e.g. 20 plates with 10 parents, 20 with 50, 20 with 100, 20 with 200, and usually a plate with the remainder of the culture. Then after one or more generations the progeny from (part of) these cultures were washed off, and again DNA was isolated from part of them (while the remainder of the culture was stored). The presence of the deletion mutant was investigated as described above, and the pool of the lowest complexity that contains the mutant of interest was investigated further. From this culture either one more rounds of "sib selection" were done, or individual animals were taken into culture, and after one generation the parents were tested for the deletion by "single worm PCR" (see Plasterk, 1995 for details).

The result is a single animal which is homozygous or possibly heterozygous for the deletion of interest. This can be studied further.

### Nematode culture.

Nematodes were cultured as described (Wood, W.B., (ed) The nematode C. elegans (Cold Spring Harbor Lab Press, Plainview)). Animals were frozen for storage as follows: Animals were grown on plates seeded with *Escherichia coli OP50*. Plates contained twice the concentration of peptone compared to the protocol given by Wood. Once cultures were cleared, 5 ml of M9 was added to the plates, and they were shaken overnight. This procedure allows starvation under fully aerobic conditions. The volume of the buffer is usually reduced to 2 ml by evaporation and absorption into the agar. For viable storage, 400 µl of each suspension was then transferred to identical positions of two microliter plates and 400 µl was added of a solution of 30% (vol/vol) glycerol in S-basal medium. Filled trays were slowly cooled and stored at -80^{o}C. Survival was monitored by thawing sample cultures 1 day after freezing and inspecting the progeny. The average number of survivors was 300; in the rare cases in which survival of one single culture in a series was <100, the complete series was discarded.
Duplicate libraries were stored in two different freezers at -80^{o}C. One compartment of such a freezer (60 x 40 x 20 cm) contains at present 5000 cultures and could contain 15,000.

## Claims

1. A method for producing a defined mutant individual from a parent organism whereby the parent organism is mutagenised and its offspring is selected for the presence of a deletion in at least one known gene.

2. A method according to claim 1 whereby the parent organism is a multi-cellular organism of which at least one cell is mutagenized.

3. A method according to claim 2 wherein the parent organism is a plant.

4. A method according to claim 2 wherein the parent organism is an animal.

5. A method according to claim 2 wherein the parent organism is a stem cell capable of replicating in culture.

6. A method according to claim 3 wherein the plant is belonging to the genus to which *Arabidopsis thaliana* belongs.

7. A method according to claim 4 wherein the animal is invertebrate.

8. A method according to claim 7 wherein the animal is selected from any of the group of insects or annelids or nematodes.

9. A method according to claim 8 wherein the animal is belonging to the genera to which *Drosophila melanogaster* or *Caenorhabditis elegans* belong.

10. A method according to any of claims 1 to 9 whereby the parent organism is mutagenised via chemical mutagenesis.

11. A method according to any of claims 1 to 9 whereby the parent organism is mutagenised via physical mutagenesis.

12. A method according to claim 10 whereby the parent organism is mutagenised via ethyl-methane-sulphonate treatment.

13. A method according to claim 10 and 11 whereby the parent organism is mutagenised via trimethyl psoralene/ultraviolet light treatment.

14. A method according to any of claims 1 to 13 whereby the offspring is selected via selective amplification of at least a part of a known gene present in siblings of the mutant individual.

15. A method according to claim 13 whereby the offspring is selected via polymerase chain reaction.

16. A method according to any of claims 1 to 13 whereby offspring is selected via hybridisation of at least a part of a known gene present in siblings of the mutant individual.

17. A method according to claim 15 whereby the hybridisation is Southern blotting or *in situ* hybridisation.

18. Use of a method according to any of claims 1 to 16 for producing mutant individuals.

19. A mutant individual obtainable by a method according to any of claims 1 to 16, or obtainable by the use according to claim 17.

20. Use of a mutant individual according to claim 18 to determine the function or importance of a gene.
